Europäisches Patentamt

European Patent Office

Office européen des brevets

(19)

(11) Numéro de publication : **0 452 210 A1**

# (12) DEMANDE DE BREVET EUROPEEN

(21) Numéro de dépôt : **91400953.5**

(22) Date de dépôt : **09.04.91**

(51) Int. Cl.$^5$ : **A61K 31/205, // (A61K31/205, 31:195)**

(30) Priorité : **09.04.90 FR 9004541**

(43) Date de publication de la demande :
**16.10.91 Bulletin 91/42**

(84) Etats contractants désignés :
**AT BE CH DE DK FR GB IT LI LU NL SE**

(71) Demandeur : **GROUPE D'ETUDES ET DE RECHERCHES DES RISQUES ENDOBIOLOGIQUES**
**914 Avenue de M.Teste - 14, le Parc**
**F-34070 Montpellier (FR)**

(72) Inventeur : **Reynier, Michel**
**14, Le Parc, 914 Avenue de M Teste**
**F-34070 Montpellier (FR)**

(74) Mandataire : **CABINET GEFIB**
**59 Rue Edouart Vaillant**
**92300 Levallois Perret (FR)**

(54) **Compositions pharmaceutiques et/ou diététiques contenant de la carnitine, de la lysine et de la méthionine avec ou sans acide pantothénique.**

(57)    La présente invention concerne des compositions pharmaceutiques et/ou diététiques, caractérisées en ce qu'elles contiennent de la carnitine en mélange avec les acides aminés précurseurs de sa synthèse endogène associée éventuellement à de l'acide pantothénique, précurseur du Co-enzyme A auquel la carnitine est étroitement associée dans son activité biologique.

Cette combinaison présente l'avantage de prolonger et d'amplifier l'activité biologique de la carnitine et permet d'obtenir une activité équivalente avec des doses plus faibles de celle-ci.

EP 0 452 210 A1

La présente invention concerne de nouvelles compositions contenant de la L-Carnitine, de la L-lysine et de la méthionine, avec ou sans acide pantothénique (Vitamine B5).

Il est connu que la carnitine et ses sels sont utilisés dans plusieurs domaines thérapeutiques, tels que les troubles des fonctions musculaires ou myocardiques rencontrées dans les déficits primaires ou secondaires en carnitine, dans les états de dénutrition post-opératoire, post-infection sévère, post-chimiothérapie, chez le diabétique, chez l'hémodialysé ou au cours d'efforts anormalement intenses.

On sait que les propriétés biologiques de la Carnitine reposent sur le rôle qu'elle exerce au niveau du métabolisme cellulaire en étroite relation avec le co-enzyme A. Ce rôle comporte un double aspect :
- rôle de transport des acides gras à longue chaine (>C10) au niveau des mitochondries où ils subissent la $\beta$-oxydation et participent aux processus énergétiques assurant la production d'ATP .

Ce rôle s' accompagne d'une faible consommation de carnitine car celle-ci est recyclée.
- rôle d'épuration d'un excès d'acides organiques intracellulaires liés au co-enzyme A ( CoA-SH), ce qui permet de diminuer les concentrations toxiques d'acyl-CoA et de maintenir un taux de CoA-SH libre compatible avec un fonctionnement mitochondrial et cellulaire normal. Ce rôle entraine une augmentation de la consommation de la Carnitine car il aboutit à la formation d' acyl-carnitines qui sont eliminés par voie urinaire.

La Carnitine intervient donc comme un facteur essentiel dans les processus énergétiques cellulaires, notamment dans les tissus riches en mitochondries, tels que les muscles et le myocarde.

Son rôle dans le métabolisme des groupements acyle est étroitement associé à celui du co-enzyme A, dont l'acide pantothénique ( Vitamine B5) est le précurseur.

D'une manière générale, les propriétés biologiques et pharmacologiques de la carnitine ont été exposées dans les revues générales suivantes :
- BAHL J.J et BRESSLER R. (1987) : The pharmacology of Carnitine
Ann. Rev. Pharmacol. Toxicol., 1987, 27, 257-277
- BORUM P.R (1983) : Carnitine
Ann. Rév. Nutr., 1983, 3, 233-259
- BREMER J (1983) : Carnitine : Métabolism and Functions
Physiol. Rév., 1983, 63, 1420-1480.

La carnitine qui est présente dans les tissus résulte, d'une part, de l'apport exogène par l'alimentation et, d'autre part, du processus de synthèse endogène au niveau du foie et du rein à partir de la lysine et de la méthionine.

Le squelette carboné de la carnitine provient de la lysine qui, dans la plupart des tissus, peut être méthylée, en présence de S-adénosyl-méthionine ; celle-ci est transformée en N-triméthyllysine, puis successivement, en $\beta$-hydroxy-N-triméthyllysine et $\gamma$-butyrobétaïne aldhéhyde et enfin, en $\gamma$-butyrobétaïne.

L'étape terminale qui transforme la $\gamma$-butyrobétaïne en L-carnitine ne s'effectue qu'au niveau du foie et du rein (cf. TAO et YOSHIMURA, J. Parenter. Enterl. Nut., 1980, 4, 469-486 et BORUM, 1983 et BORUM & FISHER, Rapport du contrat SDA 223-79-2275, Life Sciences Research Office Bethesda, 1983, 1-58 et BROQUIST, Fed. Proc. Am. Soc. Exp. Biol, 1982, 41, 2840-2842).

La présente invention concerne plus particulièrement de nouvelles compositions phamaceutiques et/ou diététiques caractérisées en ce qu'elles contiennent les acides aminés précurseurs de la synthèse endogène de carnitine (lysine et méthionine) avec ou sans le précurseur (acide pantothénique ou vitamine B5) du co-enzyme A qui est le co-facteur associé à la carnitine dans le transport des groupements acyle.

L'apport conjoint de la carnitine et de ses deux acides aminés précurseurs est de nature à renforcer l'activité biologique et l'efficacité énergétique de la carnitine. En effet, cet apport conjoint permet d'assurer une disponibilité immédiate de carnitine qui est prolongée et amplifiée par l'apport subséquent de carnitine résultant de la synthèse endogène à partir de la lysine et de la méthionine.

Ce mécanisme accroit la biodisponibilité globale de la carnitine au niveau des organes-cibles (muscles) car il a pour effet, d'une part, de diminuer les concentrations sanguines de carnitine, réduisant ainsi la fuite urinaire, d'autre part, d'instaurer une cinétique de distribution de type instantané-retard qui induit un meilleur rendement de la captation tissulaire de carnitine par les organes-cibles.

Il est important de souligner le caractère essentiel de la lysine et de la méthionine. Ces deux acides aminés sont des facteurs déterminants de la valeur nutritionnelle des protéines. Celle-ci peut être évaluée par le coefficient d'efficacité protéique qui est corrélé avec la teneur en méthionine et en lysine, comme le montre le graphique I établi à partir de données rapportées par la littérature ( cf. CHEFTEL et coll : Protéinés alimentaires - Ed. LAVOISIER, TEC et DOC, PARIS, 1985, pp 104-105 ).

La corrélation ainsi observée avec ces deux acides aminés est probablement liée à leur rôle de précurseurs de la carnitine dont l'effet positif sur le métabolisme protidique a été préalablement mis en évidence.

L'addition d'acide pantothénique (Vitamine B5) dans les préparations pharmaceutiques et/ou diététiques à base de carnitine, complète et renforce la disponibilité tissulaire des co-facteurs carnitine et co-enzyme A- qui sont impliqués dans le métabolisme des groupements acyle.

L'ensemble de tous ces éléments additionnels, d'une part, apporte aux produits diététiques et de régime à base de carnitine une efficacité énergétique renforcée et aux produits pharmaceutiques à base de carnitine une activité thérapeutique potentialisée et, d'autre part, permet ainsi d'obtenir une activité équivalente avec des doses plus faibles de carnitine.

Conformément à la législation sur les produits diététiques, les préparations peuvent contenir, en outre, de la thiamine (vitamine B1) aux doses règlementaires.

L'invention a donc spécifiquement pour objet une association à des fins pharmaceutiques, diététiques ou alimentaires qui comprend de la carnitine, de la lysine et de la méthionine en mélange ou en association avec un diluant ou un véhicule inerte adapté pour l'administration par voie digestive.

Cette association est constituée desdits trois dérivés aminés sous forme racémique ou sous forme optiquement active. Lorsque ceux-ci sont sous forme optiquement active, ils sont de préférence sous forme L- qui est la configuration naturelle.

La carnitine est employée sous forme de base mais elle peut également se trouver sous forme de sel d'addition avec un acide minéral ou organique.

La lysine peut, en outre, être incorporée au mélange sous forme de sel d'addition avec un acide minéral ou organique et tout spécialement un acide dépourvu de toxicité propre.

L'association de ces dérivés aminés peut, en outre, inclure de l'acide pantothénique (Vitamine B5) ou un de ses dérivés comme un sel de base minérale ou organique et, en particulier, le sel de sodium, le sel de potassium ou le sel de calcium.

Dans cette association, les proportions des différents constituants peuvent varier dans de larges proportions. Les proportions de carnitine et de lysine peuvent être comprises entre 1:0,1 et 1:5 exprimées en moles.

Les rapports molaires Carnitine/Methionine varient de 1:0,1 à 1:5.

Lorsque de l'acide pantothénique ou un de ses dérivés est présent, le rapport molaire carnitine/acide pantothénique peut varier de 1;0,01 à 1:0,5.

L'association de ces différents principes aminés est ensuite diluée par des excipients ou diluants inertes comme du Mannitol, du saccharose, de la caséine ou un de ses sels, des maltodextrines, du lactose, du carbonate de calcium ou du phosphate de magnésium. On peut y ajouter des agents édulcorants comme de la saccharine ou de l'Aspartam, des agents aromatisants comme l'arome orange ou l'arome citron, des agents de sapidité comme l'acide citrique, des agents épaississants ou gonflants comme des alcoylcelluloses, la carboxyméthylcellulose, des gommes végétales comme la gomme Guar ou la gomme arabique, des gommes bactériennes comme la gomme xanthane, le dextran et ses dérivés.

Elles sont présentées sous forme de gélules pour complément alimentaire des régimes hypoglucidiques, hypocaloriques ou hyperprotidiques.

Lorsque l'association est destinée à être bue, elle sera dispersée ou dissoute dans un véhicule aqueux. On peut également préparer des poudres ou des granulés contenant cette association et les dissoudre dans de l'eau de boisson au moment de l'emploi.

Il est également possible de préparer un lyophilisat par cryodessication d'une solution aqueuse en présence ou en l'absence de lécithine.

Les préparations sèches ainsi réalisées se redissolvent très aisément dans l'eau. Ces compositions sont utilisées en vue de la réalisation de médicaments, dépourvus de glucides, destinés au traitement des myalgies d'effort et des perturbations liées aux modifications électrophysiologiques musculaires ou la préparation de produits diététiques de l'effort d'apport glucidique.

Les exemples suivants illustrent l'invention sans toutefois la limiter :

EXEMPLE 1

Solution buvable

| | |
|---|---|
| L-carnitine base ......... | 0,250 g |
| L-lysine (chlorhydrate) .. | 0,135 g |
| DL- ou L-méthionine ...... | 0,050 g |
| Acide pantothénique (Vit.B5) | 0,005 g |
| Saccharinate Na .......... | 0,003 g |
| Arôme orange ............. | 0,040 g |
| Acide chlorhydrique qsp... | pH 4,5 |
| Eau purifiée qsp ......... | 10 ml |

EXEMPLE II

Lyophilisat oral

| | |
|---|---|
| L-carnitine base ......... | 0,250 g |
| L-lysine (chlorhydrate) .. | 0,135 g |
| DL- ou L-méthionine ...... | 0,050 g |
| Acide pantothénique (Vit.B5) | 0,005 g |
| Dextran 70 ............... | 0,005 g |
| Gomme xanthane ........... | 0,005 g |
| Arôme orange ............. | 0,020 g |
| Acide citrique ........... | 0,005 g |
| Saccharinate Na .......... | 0,003 g |
| Mannitol ................. | 0,400 g |
| Eau ..................... | 0,500 g |
| | pour un comprimé lyophilisé |

EXEMPLE III

Gélules

| | |
|---|---|
| L-carnitine base.......... | 250 g |
| L-lysine (chlorhydrate) .. | 135 g |
| DL- méthionine............ | 50 g |
| Acide pantothénique ...... | 5 g |
| Acide citrique ........... | 10 g |
| | pour 1.000 gélules |

EXEMPLE IV

Sachets de poudre

4

```
L-carnitine base ........    250 g
L-lysine (chlorhydrate) ..   135 g
DL- méthionine ...........     50 g
Acide pantothénique (Vit.B5)   5 g
Thiamine (Vit.B1) ........   0,030 g
Saccharose ..............   3260 g
Acide citrique ..........    100 g
Arôme orange poudre ......    200 g
        pour 1.000 sachets de poudre finis à 4 g
```

EXEMPLE V

Comprimés
Chaque comprimé renferme :

```
L-carnitine base ........    0,125 g
L-lysine (chlorhydrate) ..   0,0675 g
DL- méthionine ...........   0,025 g
Acide pantothénique (Vit.B5) 0,0025 g
Thiamine (Vit.B1) ........   0,005 mg
Saccharose ..............    0,330 g
Acide citrique ..........    0,005 g
```

EXEMPLE VI

Gélules pour complément alimentaire

```
L-carnitine base ........    125 g
L-lysine (chlorhydrate) ..   675 g
DL- ou L-méthionine ......    25 g
Acide pantothénique (Vit.B5)  2,5 g
Thiamine (Vit.B1) ........   0,005 g
Caséinate de Na ou de Ca .   230 g
Acide citrique ..........      5 g
        pour 1.000 gélules
```

EXEMPLE VII

Pâte à mâcher

```
L-carnitine base..........      250 g
L-lysine (chlorhydrate) ..      135 g
DL- ou L-méthionine ......       50 g
Acide pantothénique (Vit.B5)      5 g
Thiamine (Vt.B1) .........      0,020 g
Maltodextrine ............      500 g
Saccharose ..............       300 g
Lévulose ................       800 g
Acide citrique ..........        45 g
Citrate de K ............        7,5 g
Hydroxyde Mg ............         50 g
Arôme orange ............         2,5 g
          pour 1.000 tablettes à mâcher
```

<u>EXEMPLE VIII</u>

Granulé sec pour préparation instantanée d'une boisson, à reconstituer

```
L-carnitine base .........      0,250 g
L-lysine (chlorhydrate) ..      0,135 g
DL- ou L-méthionine ......      0,050 g
Acide pantothénique (Vit.B5)    0,005 g
Thiamine (Vit.B1) ........      0,004 mg
Saccharose ..............        9,810 g
Maltodextrine ...........        1,200 g
Lévulose ................        1,000 g
Glucose .................        1,000 g
Acide citrique ..........        0,800 g
Citrate de K ............        0,250 g
Acide ascorbique ........        0,250 g
Arôme citron ............        0,200 g
Chlorure Na ..............        0,050 g
      dans un sachet ou un petit flacon de verre
```

Boisson à reconstituer par dissolution dans un verre d'eau (environ 180 ml).

**Revendications**

1°- Compositions pharmaceutiques et/ou diététiques caractérisées en ce qu'elles contiennent de la carnitine, en association à de la lysine et de la méthionine, en mélange ou en association avec un diluant ou un véhicule inerte adapté pour l'administration par voie digestive.

2°- Compositions pharmaceutiques et/ou diététiques selon la revendication 1 carctérisées en ce qu'elles contiennent de la carnitine, en mélange avec de la lysine, de la méthionine et, en outre, de l'acide pantothénique

6

(Vitamine B5).

3°- Compositions selon l'une des revendications 1 or 2 dans lesquelles la carnitine se trouve présente sous forme de sel d'addition avec un acide minéral ou organique.

4°- Compositions selon l'une des revendications 1 à 3 dans lesquelles la lysine est sous forme de sel d'addition avec un acide minéral ou organique.

5°- Compositions selon l'une des revendications 1 à 4 dans lesquelles la lysine, la méthionine et l'acide pantothénique sont présents sous forme optiquement-active et notamment sous la forme L-.

6°- Compositions selon l'une des revendications 1 ou 2, caractérisées en ce que les proportions relatives de carnitine et de lysine sont respectivement comprises entre 1:0,1 et 1:5 exprimées en moles.

7°- Compositions selon l'une des revendications 1 ou 2 caractérisées en ce que les proportions relatives de carnitine et de méthionine sont respectivement comprises entre 1:0,1 et 1:5 exprimées en moles.

8°- Compositions selon l'une des revendications 1 et 2 caractérisées en ce que les proportions relatives de carnitine et d'acide pantothénique (Vitamine B5) sont respectivement comprises entre 1:0,01 et 1:0,5 exprimées en moles.

9°- Compositions selon l'une des revendications 1 à 5 caractérisées en ce qu'elles sont présentées sous forme compatible avec une administration digestive sous forme de poudre, de sachets, de gélules, d'ampoules buvables, de lyophilisat oral, de comprimés, de pâte à mâcher, de boisson à reconstituer.

10°- Compositions selon l'une des revendications 1 à 5 caractérisées en ce qu'elles sont présentées sous forme de solutions buvables.

11°- Compositions selon l'une des revendications 1 à 5 caractérisées en ce qu'elles sont présentées sous forme de lyophilisat oral.

12°- Compositions selon l'une des revendications 1 à 5 caractérisées en ce qu'elles sont présentées sous forme de gélules ou de comprimés.

13°- Compositions selon l'une des revendications 1 à 5 caractérisées en ce qu'elles sont présentées sous forme de poudre ou de sachet.

14°- Compositions selon l'une des revendications 1 à 5 caractérisées en ce qu'elles sont présentées sous forme de pâte à mâcher.

15°- Compositions selon l'une des revendications 1 à 5 caractérisées en ce qu'elles sont présentées sous forme de poudre pour boisson à reconstituer.

16°- Compositions selon l'une des revendications 1 à 5 caractérisées en ce qu'elles sont présentées sous forme de gélules pour complément alimentaire des régimes hypoglucidiques, hypocaloriques ou hyperprotidiques.

17°- Utilisation des compositions selon les revendications 1 à 9 en vue de la réalisation de médicaments, dépourvus de glucides, destinés au traitement des myalgies d'effort et des perturbations liées aux modifications électrophysiologiques musculaires.

18°- Utilisation des compositions selon les revendications 1 à 9 dans la préparation de produits diététiques de l'effort d'apport glucidique.

19°- Procédé de préparation d'une composition pharmaceutique et/ou diététique selon l'une des revendications 1 à 16 caractérisé en ce que l'on combine la carnitine avec la lysine ou un de ses sels d'acide organique ou minéral et la méthionine, avec ou sans acide pantothénique, et en ce que l'on mélange cette combinaison avec des excipients pharmaceutiquement et/ou diététiquement inertes.

Office européen
des brevets

**RAPPORT DE RECHERCHE EUROPEENNE**

Numero de la demande

EP 91 40 0953

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 354 848 (LABORATOIRE ROGER BELLON) <br> * Pages 19-21; revendications 1-15 * <br> --- | 1-19 | A 61 K 31/205 // <br> (A 61 K 31/205 <br> A 61 K 31:195) |
| X | UNLISTED DRUGS, vol. 23, no. 1, janvier 1971, Chatham, New Jersey, US <br> * Page 9d: "OSVICAL-LISINA" * <br> --- | 1-19 | |
| X | UNLISTED DRUGS, vol. 23, no. 3, mars 1971, Chatham, New Jersey, US <br> * Page 41n: "REGENERON" * <br> ----- | 1-19 | |

| DOMAINES TECHNIQUES RECHERCHES (Int. Cl.5) |
|---|
| A 61 K |

Le présent rapport a été établi pour toutes les revendications

| Lieu de la recherche | Date d'achèvement de la recherche | Examinateur |
|---|---|---|
| LA HAYE | 23-05-1991 | BRINKMANN C. |